(19) Europäisches Patentamt
European Patent Office
Office européen des brevets

(11) Veröffentlichungsnummer : **0 106 038**
**B1**

(12) **EUROPÄISCHE PATENTSCHRIFT**

(45) Veröffentlichungstag der Patentschrift : **15.05.85**

(51) Int. Cl.⁴ : **C 07 C118/00**, C 01 C 3/14

(21) Anmeldenummer : **83107372.1**

(22) Anmeldetag : **27.07.83**

(54) **Verfahren zur Herstellung von Isocyansäure oder aliphatischer Isocyanate durch oxidative Dehydrierung von Formamiden.**

(30) Priorität : **06.08.82 DE 3229323**

(43) Veröffentlichungstag der Anmeldung : **25.04.84 Patentblatt 84/17**

(45) Bekanntmachung des Hinweises auf die Patenterteilung : **15.05.85 Patentblatt 85/20**

(84) Benannte Vertragsstaaten : **BE DE FR GB IT NL**

(56) Entgegenhaltungen : **EP-A- 0 000 602**

(73) Patentinhaber : **BASF Aktiengesellschaft**
**Carl-Bosch-Strasse 38**
**D-6700 Ludwigshafen (DE)**

(72) Erfinder : **Halbritter, Klaus, Dr.**
**Schwarzwaldstrasse 19**
**D-6800 Mannheim 1 (DE)**
Erfinder : **Eggersdorfer, Manfred, Dr.**
**Weinbietstrasse 22**
**D-6718 Gruenstadt (DE)**
Erfinder : **Brenner, Karl**
**Riedsaumstrasse 40**
**D-6700 Ludwigshafen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 106 038 B1

**Beschreibung**

Die Erfindung betrifft ein Verfahren zur Herstellung von Isocyansäure oder aliphatischer Isocyanate durch Oxidation von Formamiden in der Gasphase in Gegenwart eines Kupfer- oder Silberkatalysators und anschließender Kondensation der Reaktionsgase.

Monomere Isocyansäure läßt sich durch Pyrolyse von Cyanursäure, Harnstoff oder von Urethanen herstellen. Allerdings laufen die jeweiligen Umsetzungen nicht sehr selektiv ab und liefern in der Regel Isocyansäure, die mit anderen Pyrolyseprodukten verunreinigt ist. Zur weiteren Reinigung sind aufwendige und teilweise gefährliche Reinigungsoperationen nötig (Chem. Soc. Rev. *11*, 41 (1982)).

Verfahren, die beispielsweise von Methan, Kohlenmonoxid oder Acetonitril — also von Produkten mit niedriger Oxidationsstufe — ausgehen, erfordern aufgrund der drastischen Reaktionsbedingungen (Temperaturen von 800 bis 1 200 °C) einen großen technischen Aufwand und führen häufig nur zu geringen Umsätzen (JP-A-42 799/1974, JP-A-3999/1975 ; JP-A-125 999/1978).

Die Freisetzung von Isocyansäure aus ihren Alkali- oder Erdalkalisalzen mittels Säuren gelingt nur unter besonderen Reaktionsbedingungen, jedoch ist die Ausbeute wegen der Hydrolyse des Isocyanats bzw. der Isocyansäure und durch das Zusammenbacken der meist schwerlöslichen Ausgangsmaterialien unbefriedigend (Chem. Soc. Rev. *11*, 41 (1982)).

Aus Z. Chem. *14*, 192 (1974) ist bekannt, daß substituierte Formamide katalytisch zu Isocyanaten dehydriert werden können. In der Publikation wird aufgezeigt, daß bei den zur Reaktion notwendigen drastischen Bedingungen (750 bis 1 000 °C) nur etwa 14 % an Isocyanat isoliert werden können.

Die EP-A-602 beschreibt die Oxidation von monosubstituierten Formamiden in der Gasphase bei Temperaturen von 300 bis 600 °C in Gegenwart von Luft oder eines Sauerstoff-enthaltenden Gases an Katalysatoren der Gruppe IB oder VIII. Beste Ergebnisse liefert nach der Patentschrift die Verwendung eines Silber-Katalysators in Form von Silber-Wolle. Zweckmäßigerweise wird das Formamid zusammen mit einem inerten Lösungsmittel dem Reaktor zugeführt. Um gute Selektivitäten zu erzielen, wird dem zu dehydrierenden Gemisch eine kleine Menge Halogen- oder Schwefelverbindung beigefügt. Nach dem Verfahren werden die Reaktionsgase abgekühlt und die Wasserphase von der Isocyanat-Phase separiert. Andere Möglichkeiten der Wasserentfernung aus dem Reaktionsgemisch werden aufgezeigt.

Das Verfahren kann Ausbeuten bis maximal 85 % liefern ; dann aber liegen die Raum-Zeit-Ausbeuten nur bei 0,07 kg Isocyanat/l Katalysatorvolumen und Stunde und kleiner, oder aber bei größeren Belastungen sinkt die Ausbeute auf etwa 32 %, wenn die Raum-Zeit-Ausbeute auf 0,72 kg Isocyanat/l Katalysatorvolumen und Stunde angehoben wird. Sowohl der Umsatz als auch die Selektivität sind bei höheren Belastungen unbefriedigend und führen zu komplexen Austragsgemischen.

Das Verfahren ist deshalb mit Bezug auf einfachen und wirtschaftlichen Betrieb zusammen mit guter Ausbeute, Raum-Zeit-Ausbeute und Reinheit des Endstoffes noch nicht befriedigend.

Zudem war zu erwarten, daß es beim Einsatz von unsubstituiertem Formamid unter diesen Bedingungen zu thermischen Zerfallsreaktionen des Formamids kommen würde (Z. anorg. Chem. *262*, 14 (1950)) ; Mh. Chem. *86*, 671 (1955)).

Es wurde nun gefunden, daß man Isocyansäure oder aliphatische Isocyanate durch Oxidation von Formamiden der Formel

R—NH—CHO

in der R ein Wasserstoffatom oder einen Alkylrest mit 1 bis 8 Kohlenstoffatomen bedeutet, mit Sauerstoff oder einem Sauerstoff enthaltenden Gas in der Gasphase über einem Mehrschichten-Katalysator aus Kupfer- oder Silberkristallen und anschließendes schnelles Abkühlen vorteilhaft herstellt, wenn man als Katalysator Kupfer- oder Silberkristalle der Korngröße 0,1 bis 5,0 mm, geschichtet nach Korngröße verwendet, wobei die Korngröße in den oberen Lagen geringer ist und man die durchschnittliche Verweilzeit der Moleküle zwischen 0,000 5 bis 0,1 Sekunden und die Reaktionstemperatur zwischen 300 und 700 °C wählt.

Die Umsetzung kann durch folgende Formel wiedergegeben werden :

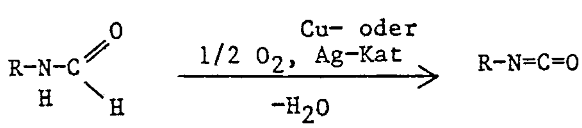


Im Vergleich zu dem bekannten Verfahren liefert das Verfahren nach der Erfindung überraschend auf einfacherem und wirtschaftlicherem Wege ein besseres Gesamtergebnis mit Bezug auf Ausbeute, Raum-Zeit-Ausbeute und Reinheit des Endstoffes.

Für das Verfahren geeignete Ausgangsstoffe sind neben dem Grundkörper Formamid monosubstituierte Formamide, in denen R einen Alkylrest mit 1 bis 8 C-Atomen bedeutet. Das jeweilige Formamid wird in Dampfform, vorzugsweise lösungsmittelfrei, dem Reaktionsraum zugeführt. Gegebenenfalls kann die Zufuhr auch in Gegenwart eines inerten Lösungsmittels, wie Tetrahydrofuran, Dioxan, Toluol oder Hexan

erfolgen. Das Gewichtsverhältnis Lösungsmittel : Formamid beträgt dann vorteilhaft 0,5 : 1 bis 4 : 1.

Sowohl bei der lösungsmittelfreien Variante, als auch beim Arbeiten mit Lösungsmittel ist die Abmischung mit einem Inertgas vorteilhaft. Als unter den Reaktionsbedingungen inerte Gase (Inertgas) werden zweckmäßig Edelgase wie Xenon, Argon, Neon, Helium, bevorzugt Stickstoff, Kohlenoxid und/oder Kohlendioxid verwendet. Das Inertgas kann für sich allein oder im Gemisch mit Luft eingesetzt werden. Das Molverhältnis von Inertgas zu Sauerstoff beträgt in der Regel mindestens 4,4, zweckmäßig 4,4 bis 20 : 1, wobei sich die Angaben bezüglich Inertgas stets auf die Gesamtmenge, d. h. des Inertgasanteils der bevorzugt verwendeten Luft beziehen.

Als oxidierendes Agens lassen sich sowohl der reine Sauerstoff als auch Sauerstoff enthaltende Gase, insbesondere Luft, verwenden. Sauerstoff, in der Regel in Gestalt von Luft und Formamid werden zweckmäßig im Molverhältnis von 0,2 bis 1,0 insbesondere 0,3 bis 0,7 Mol Sauerstoff je Mol Formamid angewandt.

Die Verweilzeit des Gasgemisches im Reaktionsraum beträgt 0,000 5 bis 0,1, bevorzugt 0,001 bis 0,05 Sekunden. Aufgrund der kurz gewählten Verweilzeit können die Nebenreaktionen fast vollständig unterdrückt werden, obwohl das Ausgangsprodukt quantitativ umgesetzt wird.

Als Katalysatoren sind Kupfer- oder Silberkristalle geeignet, wobei für die Herstellung der aliphatischen Isocyanate Silber-Katalysatoren bevorzugt sind.

Die Gesamtschichtdicke des Katalysators beträgt zweckmäßig 10 bis 40, vorzugsweise 15 bis 30 Millimeter. Die Katalysatorteilchen werden in Schichten nach Korngröße auf ein Netz aus Silber oder Edelstahl in den vertikal aufgestellten Reaktor gegeben. Bei einem 2-Schichten-Katalysator enthält die obere Teilschicht 20 bis 50 Gewichtsprozent des Katalysators mit Teilchen der Korngröße 0,1 bis 0,75 Millimeter, die untere Schicht 50 bis 80 Gewichtsprozent des Katalysators. Bei der Umsetzung von unsubstituiertem Formamid beträgt die Korngröße in der Regel hier 0,75 bis 5,0 mm, während bei der Umsetzung der substituierten Formamide insbesondere Korngrößen im Bereich von 0,75 bis 2,5 mm Verwendung finden.

Bei einem 3-Schichten-Katalysator befinden sich im oberen Teil 5 bis 25 Gewichtsprozente, im mittleren Teil 25 bis 70 Gewichtsprozente und im unteren Teil 15 bis 40 Gewichtsprozent der Katalysatorkörner. Die obere Schicht enthält vorteilhaft Korngrößen von 0,1 bis 0,4 Millimeter, die mittlere von 0,4 bis 0,75 Millimeter und die untere Schicht von 0,75 bis 5,0 Millimeter (für unsubstituiertes Formamid) bzw. 0,75 bis 2,5 Millimeter (für substituiertes Formamid). Gleichermaßen ist ein Katalysatorbett mit mehr als 3, z. B. 4 oder 5, Schichten geeignet.

Die Standzeit des Katalysators mit konstanter Aktivität und Selektivität beträgt 3 Monate und mehr. Der Katalysator wird zweckmäßigerweise mit 0,2 bis 2 t, insbesondere 0,3 bis 1,5 t Formamid je $m^2$ Katalysatorquerschnitt und Stunde belastet. Die Raum-Zeit-Ausbeuten betragen 15 bis 60 kg Isocyansäure bzw. aliphatisches Isocyanat pro Liter Reaktorvolumen und Stunde und liegen um den Faktor 20 bis 100 höher als man im Hinblick auf die EP-A 602 hätte erwarten können.

Für die oxidative Dehydrierung leitet man das Gasgemisch aus jeweiligem Formamid, Luft und Inertgas in den vorgenannten Mengen bei Temperaturen von 300 bis 700 °C durch den Katalysator. Der für die Herstellung von Isocyansäure bevorzugte Temperaturbereich liegt bei 520 bis 650 °C, während für die aliphatischen Isocyanate der Temperaturbereich von 500 bis 700 °C, insbesondere von 550 bis 650 °C, bevorzugt sind. Die Reaktion wird im allgemeinen bei Drücken zwischen 0,8 und 2 bar, vorzugsweise 1,05 bis 1,5 bar, kontinuierlich durchgeführt.

Die aus der letzten Katalysatorschicht austretenden 300 bis 700 °C heißen Reaktionsgase werden im Gleichstrom mit einem inerten Lösungsmittel oder dem Isocyansäure- bzw. Isocyanat-enthaltenden Lösungsmittelgemisch rasch auf − 20 bis − 30 °C (bei der Herstellung von Isocyansäure) bzw. 5 bis 15 °C (bei der Herstellung von aliphatischen Isocyanaten) abgekühlt. Als Lösungsmittel wählt man ein mit der Isocyansäure bzw. den Isocyanaten mischbares, mit Wasser 2-Phasen bildendes Lösungsmittel aus der Verbindungsklasse der Kohlenwasserstoffe, substituierten Kohlenwasserstoffe, Ether oder im Falle der Isocyanate auch der Ketone, wie Heptan, Toluol, Methylenchlorid oder Methyl-tert.-butylether. Das Gemisch wird zusammen mit den Reaktionsgasen über eine mit Glasringen gefüllte Säule zum vollständigen Lösen der Isocyansäure bzw. des Isocyanates im Lösungsmittel geführt. Die mittlere Verweilzeit der Mischung des Reaktionsgemisches mit dem Lösungsmittel in der gefüllten Glassäule beträgt 0,1 bis 5 Sekunden. Im nachfolgenden Phasenseparationsgefäß trennen sich die wäßrige und die Isocyansäure- bzw. Isocyanat-enthaltende Lösungsmittel-Phase. Eventuelle Restmengen von unumgesetztem Formamid und durch Hydrolyse von Isocyansäure bzw. Isocyanat gebildete Spaltprodukte verbleiben in der wäßrigen Phase und werden von der Isocyansäure- bzw. Isocyanat-Lösung abgetrennt. Der Anteil der Hydrolyseprodukte bleibt unter 1 % bezogen auf gebildete Isocyansäure bzw. Isocyanat. Die organische Phase wird über einen Wärmeaustauscher gekühlt und den Reaktionsgasen entgegengeführt. Die Lösungen enthalten 5 bis 30 Gewichts-Prozent an Isocyansäure bzw. Isocyanat.

Durch die Wahl der Quenchflüssigkeit, der Temperatur und der Menge Quenchflüssigkeit können die kondensierbaren Anteile vollständig gewonnen werden.

Die erhaltenen Lösungen der monomeren Isocyansäure können unmittelbar für organische Synthesen Verwendung finden, so daß sich eine Isolierung der monomeren Isocyansäure erübrigt. Da monomere Isocyansäure bei Raumtemperatur in Lösungen erheblich stabiler als in reiner Form ist, ergibt das erfindungsgemäße Verfahren einen besonderen technischen Vorteil bezüglich Einfachheit, Ungefährlichkeit und Wirtschaftlichkeit, wobei überraschend ist, daß die gebildete Isocyansäure unter den Reaktionsbedingungen nicht hydrolysiert wird.

Die Lösungen der aliphatischen Isocyanate werden im allgemeinen direkt weiterverwendet oder das Isocyanat wird durch Destillation vom Lösungsmittel abgetrennt. Das anfallende Lösungsmittel wird wieder zum Quenchen oder falls notwendig zur Abgaswäsche zurückgeführt.

Beispiel 1

Herstellung von n-Butylisocyanat

Man verwendet eine Anlage (siehe Zeichnung) mit Zuführungen für Luft und Inertgase (1) sowie für Formamid (2) auf den Kopf eines senkrecht stehenden Rohrreaktors (3). Der Rohrreaktor enthält eine Silberkatalysatorschicht folgender Zusammensetzung :

| | Anteil am Katalysator Gew. % | Korngröße mm |
|---|---|---|
| Schicht 1 | 20 | 0,2 - 0,4 |
| Schicht 2 | 50 | 0,4 - 0,75 |
| Schicht 3 | 30 | 0,75 - 1,0 |

Der Reaktor geht über in den Quenchraum der den Kopf einer Füllkörperkolonne (4) bildet. Daran schließt sich ein Phasentrenngefäß (5) an. Die organische Phase wird als Quenchflüssigkeit mit einer Pumpe (7) über einen Wärmetauscher (8) und eine Düse unmittelbar hinter der Katalysatorschicht in die heißen Gase gesprüht, die im Quenchraum auf 5 bis 10 °C abgekühlt und kondensiert bzw. gelöst werden. Das Abgas wird über eine weitere Füllkörperkolonne (10) geführt mit aus der Lösungsmittelrückführung kommendem Lösungsmittel (11) aus Kolonne (12) gewaschen und über die Leitung (13) zur Verbrennung geführt. Nach Abtrennung des Lösungsmittels in der Kolonne (12) wird das Produkt über die Leitung (14) erhalten.

Dem Reaktor werden pro Stunde ein Gemisch von 155 kg n-Butyl-Formamid und 120 $m^3$ Luft mit einer Eintrittstemperatur von 270 °C zugeführt. Die Reaktionstemperatur beträgt 630 °C, die durchschnittliche Verweilzeit im Katalysatorraum liegt bei $37 \cdot 10^{-3}$ Sekunden.

Die in den Quenchraum eintretenden Reaktionsgase werden mit Isocyanat-enthaltenden Lösungsmittel auf 5 bis 10 °C abgekühlt. Die Ausbeute an n-Butyl-Isocyanat beträgt 89 % der Theorie, der Umsatz errechnet sich zu 99 %, die Raumzeit-Ausbeute 15 g Isocyanat pro $cm^3$ Katalysatorvolumen und Stunde.

Beispiel 2

Herstellung von Isocyansäure

Man verwendet den in Beispiel 1 beschriebenen Reaktor. Die Korngröße des Silberkatalysators beträgt aber in Schicht 3 jetzt 0,75 bis 5,0 mm.

Man arbeitet analog Beispiel 1, kühlt jedoch die heißen Gase im Quenchraum auf − 20 bis 30 °C ab.

Dem Reaktor werden pro Stunde ein Gemisch von 450 g Formamid und 260 l Luft mit einer Eintrittstemperatur von 270 °C zugeführt. Die Reaktionstemperatur beträgt 540 °C, die durchschnittliche Verweilzeit im Katalysatorbett liegt bei 0,02 Sekunden.

Die in den Quenchraum eintretenden Reaktionsgase werden mit Isocyansäure-enthaltendem Lösungsmittel auf − 20 °C abgekühlt.

Die gebildete monomere Isocyansäure wird zur quantitativen Bestimmung mit einem Überschuß an Schwefelsäure hydrolysiert und das gebildete $NH_3$ rücktitriert. Die Ausbeute an Isocyansäure beträgt 85 % der Theorie, der Umsatz errechnet sich zu 99 %. Die Raum-Zeit-Ausbeute beträgt 18 g Isocyansäure pro $cm^3$ Katalysatorvolumen und Stunde.

Diese Rohlösung von Isocyansäure kann unmittelbar für organische Synthesen Verwendung finden.

Beispiel 3

Herstellung von n-Butylharnstoff

In diesem Beispiel wird die entstehende Isocyansäure direkt mit n-Butylamin umgesetzt.

Man arbeitet dabei analog Beispiel 2. Anstelle des Silberkatalysators wird jedoch ein Kupferkatalysator mit gleicher Korngröße eingesetzt.

Im Quenchkreislauf werden n-Butylamin in Wasser vorgelegt (Gewichtsverhältnis n-Butylamin : Wasser = 3 : 1).

Dem Reaktor werden analog Beispiel 2 pro Stunde 450 g Formamid und 260 l Luft zugeführt.

Zur Aufarbeitung wird aus der erhaltenen Lösung Wasser verdampft, der gebildete n-Butylharnstoff fällt dabei analysenrein an (Ausbeute 80 % d. Th.).

**Patentansprüche**

1. Verfahren zur Herstellung von Isocyansäure oder aliphatischer Isocyanate durch Umsetzung von Formamiden der Formel

R—NH—CHO

in der R ein Wasserstoffatom oder einen Alkylrest mit 1 bis 8 Kohlenstoffatomen bedeutet, mit Sauerstoff oder einem Sauerstoff enthaltenden Gas in der Gasphase über einem Katalysator aus Kupfer- oder Silberkristallen und anschließendes schnelles Abkühlen, dadurch gekennzeichnet, daß man die Umsetzung bei Temperaturen von 300 bis 700 °C, einer mittleren Verweilzeit von 0,1 bis 0,000 5 sec. und über einem Mehrschichten-Katalysator mit einer Korngröße der Kupfer- oder Silberkristalle von 0,1 bis 5,0 mm durchführt, wobei die Kupfer- oder Silberkristalle nach Korngrößen so geschichtet sind, daß die Korngröße in der oberen Lage bzw. den oberen Lagen geringer ist.

2. Verfahren gemäß Anspruch 1, dadurch gekennzeichnet, daß man die Oxidation lösungsmittelfrei durchführt.

3. Verfahren gemäß Anspruch 1, dadurch gekennzeichnet, daß man die Umsetzung der Formamide, die einen Alkylrest besitzen, bei einer Temperatur von 500 bis 700 °C über einem Silberkatalysator mit einer Korngröße der Silberkristalle von 0,1 bis 2,5 mm durchführt.

4. Verfahren gemäß Anspruch 3, dadurch gekennzeichnet, daß man einen Zweischichten-Katalysator verwendet, dessen untere Schicht 50 bis 80 Gew.% des gesamten Silbers mit Teilchen der Korngröße 0,75 bis 2,5 mm und dessen obere Schicht 20 bis 50 Gew.% mit Teilchen der Korngröße 0,1 bis 0,75 mm enthält.

5. Verfahren gemäß Anspruch 3, dadurch gekennzeichnet, daß man einen Dreischichten-Katalysator verwendet, dessen untere Schicht 15 bis 40 Gew.% des gesamten Silbers mit Teilchen der Korngröße von 0,75 bis 2,5 mm, dessen mittlere Schicht 25 bis 70 Gew.% mit Teilchen der Korngröße von 0,4 bis 0,75 mm und dessen obere Schicht 5 bis 25 Gew.% mit Teilchen der Korngröße 0,1 bis 0,4 mm enthält.

6. Verfahren gemäß Anspruch 1, dadurch gekennzeichnet, daß man für die Umsetzung von unsubstituiertem Formamid einen Zweischichten-Katalysator verwendet, dessen untere Schicht 50 bis 80 Gew.% des gesamten Kupfers oder Silbers mit Teilchen der Korngröße 0,75 bis 5,0 mm und dessen obere Schicht 20 bis 50 Gew.% mit Teilchen der Korngröße 0,1 bis 0,75 mm enthält.

7. Verfahren gemäß Anspruch 1, dadurch gekennzeichnet, daß man für die Umsetzung von unsubstituiertem Formamid einen Dreischichten-Katalysator verwendet, dessen untere Schicht 15 bis 40 Gew.% des gesamten Kupfers oder Silbers mit Teilchen der Korngröße von 0,75 bis 5,0 mm, dessen mittlere Schicht 25 bis 70 Gew.% mit Teilchen der Korngröße 0,4 bis 0,75 mm und dessen obere Schicht 5 bis 25 Gew.% mit Teilchen der Korngröße 0,1 bis 0,4 mm enthält.

**Claims**

1. A process for the preparation of isocyanic acid or aliphatic isocyanates by reacting formamides of the formula

R—NH—CHO

where R is hydrogen or alkyl of 1 to 8 carbon atoms, with oxygen or an oxygen-containing gas in the gas phase over a catalyst consisting of copper or silver crystals, and subsequent rapid cooling, wherein the reaction is carried out at a temperature of from 300 to 700 °C over a catalyst which is arranged in a plurality of layers and whose copper or silver crystals have a particle size of from 0.1 to 5.0 mm, the mean residence time being from 0.000 5 to 0.1 sec, and the copper or silver crystals being so arranged according to their particle size that the particle size is smaller in the upper layer or layers.

2. A process as claimed in claim 1, wherein the oxidation is carried out in the absence of a solvent.

3. A process as claimed in claim 1, wherein the reaction of the formamides containing an alkyl radical is carried out at a temperature of from 500 to 700 °C over a silver catalyst whose crystals have a particle size of from 0.1 to 2.5 mm.

4. A process as claimed in claim 3, wherein a catalyst arranged in two layers is used, the lower and upper layers respectively containing 50 to 80 % and 20 to 50 % by weight of the total amount of silver, and respectively consisting of particles having a size of from 0.75 to 2.5 mm and from 0.1 to 0.75 mm.

5. A process as claimed in claim 3, wherein a catalyst arranged in three layers is used, the lower, middle and upper layers respectively containing 15 to 40 %, 25 to 70 % and 5 to 25 % by weight of the total amount of silver, and respectively consisting of particles having a size of from 0.75 to 2.5 mm, from 0.4 to 0.75 mm and from 0.1 to 0.4 mm.

6. A process as claimed in claim 1, wherein a catalyst arranged in two layers is used for the reaction of unsubstituted formamide, the lower and upper layers of the catalyst respectively containing 50 to 80 % and 20 to 50 % by weight of the total amount of copper or silver, and respectively consisting of particles having a size of from 0.75 to 5 mm and from 0.1 to 0.75 mm.

7. A process as claimed in claim 1, wherein a catalyst arranged in three layers is used for the reaction of unsubstituted formamide, the lower, middle and upper layers of the catalyst respectively containing 15 to 40 %, 25 to 70 % and 5 to 25 % by weight of the total amount of copper or silver, and respectively consisting of particles having a size of from 0.75 to 5 mm, from 0.4 to 0.75 mm and from 0.1 to 0.4 mm.

**Revendications**

1. Procédé de préparation de l'acide isocyanique et d'isocyanates aliphatiques par la réaction de formamides de la formule

R—NH—CHO

dans laquelle R désigne un atome d'hydrogène ou un radical alkyle en $C_1$ à $C_8$, en phase gazeuse avec l'oxygène ou un mélange gazeux contenant de l'oxygène sur un catalyseur aux cristaux de cuivre ou d'argent, suivie d'un refroidissement rapide, caractérisé en ce que la réaction est réalisée à des températures de 300 à 700 °C et avec une durée de contact moyenne entre 0,1 et 0,000 5 seconde avec un catalyseur multi-couche aux cristaux de cuivre ou d'argent d'une granulométrie de 0,1 à 5,0 mm, les cristaux de cuivre ou d'argent étant disposés de manière telle, en fonction de leur granulométrie, que la ou les couches supérieures contiennent ceux avec la granulométrie la plus réduite.

2. Procédé suivant la revendication 1, caractérisé en ce que l'oxydation est réalisée en l'absence d'un solvant.

3. Procédé suivant la revendication 1, caractérisé en ce que la réaction des formamides avec un radical alkyle est réalisée entre 500 et 700 °C sur un catalyseur à l'argent, dont les cristaux possèdent une granulométrie de 0,1 à 0,75 mm.

4. Procédé suivant la revendication 3, caractérisé en ce que l'on emploie un catalyseur à deux couches, dont la couche inférieure contient entre 50 et 80 % en poids de la totalité de l'argent et est formée de particules d'une granulométrie de 0,75 à 2,5 mm et la couche supérieure contient entre 20 et 50 % en poids et est formée de particules d'une granulométrie de 0,1 à 0,75 mm.

5. Procédé suivant la revendication 3, caractérisé en ce que l'on emploie un catalyseur à trois couches, dont la couche inférieure contient entre 15 et 40 % en poids de la totalité de l'argent et est formée de particules d'une granulométrie de 0,75 à 2,5 mm, la couche médiane 25 à 70 % en poids et des particules d'une granulométrie de 0,4 à 0,75 mm et la couche supérieure 5 à 25 % en poids et des particules d'une granulométrie de 0,1 à 0,4 mm.

6. Procédé suivant la revendication 1, caractérisé en ce que l'on emploie pour la réaction du formamide non substitué un catalyseur à deux couches, dont la couche inférieure contient 50 à 80 % en poids de la totalité du cuivre ou de l'argent et est formée de particules d'une granulométrie de 0,75 à 5,0 mm et la couche supérieure 20 à 50 % en poids et des particules d'une granulométrie de 0,1 à 0,75 mm.

7. Procédé suivant la revendication 1, caractérisé en ce que l'on emploie pour la réaction du formamide non substitué un catalyseur à trois couches, dont la couche inférieure contient 15 à 40 % en poids de la totalité du cuivre ou de l'argent et est formée de particules d'une granulométrie de 0,75 à 5,0 mm, la couche médiane 25 à 70 % en poids et des particules d'une granulométrie de 0,4 à 0,75 mm et la couche supérieure 5 à 25 % en poids et des particules d'une granulométrie de 0,1 à 0,4 mm.

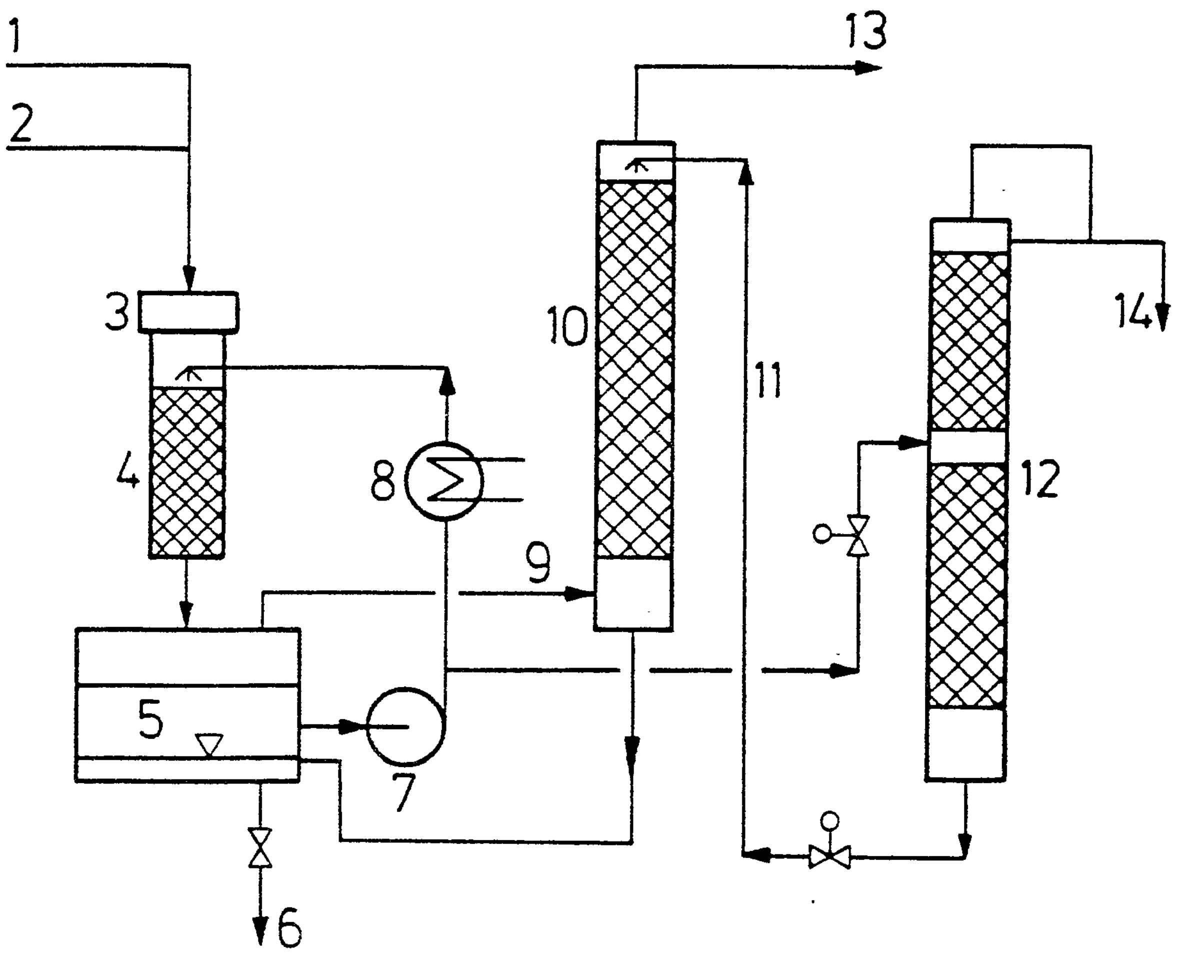
1
2
3
4
5
6
7
8
9
10
11
12
13
14